# EUROPÄISCHE PATENTSCHRIFT

(11) **EP 0 733 349 B1**
(45) Veröffentlichungstag und Bekanntmachung des Hinweises auf die Patenterteilung: **26.04.2000**
(21) Anmeldenummer: 96102498.1
(22) Anmeldetag: 20.02.1996
(51) Int. Cl.: A61F 5/02, A44B 11/20

(54) **Hyperextensionsorthese**
Hyperextension orthesis
Orthèse de hyperextension

(30) Priorität: 24.03.1995 DE 29504983 U
(43) Veröffentlichungstag der Anmeldung: 25.09.1996
(73) Patentinhaber: Otto Bock Orthopädische Industrie Besitz- und Verwaltungs-Kommanditgesellschaft, 37115 Duderstadt (DE)
(72) Erfinder: Zepf, Armin, 37115 Duderstadt (DE)
(74) Vertreter: Gramm, Werner, Prof. Dipl.-Ing.

(56) Entgegenhaltungen:
- WO-A-92/06606
- DE-C- 3 928 628
- DE-U- 8 903 944
- DE-U- 29 504 983

## Beschreibung

Die Erfindung betrifft eine Hyperextensionsorthese mit einem Verschlußkasten, der an dem freien Ende eines sich von einer Basisplatte zur Seite erstreckenden Seitensteges befestigt ist und das eine Ende eines mit einer Rückenpelotte verbundenen Taillenbandes selbsttätig verriegelnd aufnimmt, wobei der Verschlußkasten einen das eine Ende eines Federarmes bildenden Schließhaken aufweist, der beim Einschieben des Taillenbandes in einen sich durch den Verschlußkasten hindurch erstreckenden Einschubschlitz unter Federwirkung selbsttätig in eine der Rastausnehmungen im Taillenband eingreift und durch manuelles Zusammendrücken zweier quer zum Einschubschlitz verschiebbar im Verschlußkasten gelagerter Schieber gegen die Federwirkung entriegelbar ist.

Die Hyperextensionsorthese als solche läßt sich z. B. der DE 39 28 628 C1 entnehmen. Die verstellbare Verriegelung des Taillenbandes in einem Verschlußkasten ist Gegenstand einer offenkundigen Vorbenutzung.

Der WO-A-92/06606 läßt sich ein Verschlußelement für um die Hüfte zu tragende Plastikgürtel entnehmen. Das Verschlußelement weist einen aus Kunststoff bestehenden Verschlußkasten auf, der sich aus einer oberen Abdeckung, einer Bodenplatte und zwei gegenüberliegenden Längswandungen zusammensetzt. Der Verschlußkasten ist einstückig mit dem einen Ende des Plastikgürtels ausgebildet und umschließt ein eingesetztes Verriegelungselement. Letzteres weist einen einen Schließhaken tragenden Federarm auf und greift an seinem dem Schließhaken abgewandten, in Einschubrichtung des Taillenbandes vorn liegenden Endes mit einer Feder in eine Nut der Bodenplatte des Verschlußkastens formschlüssig ein. Der Schließhaken ragt von dem Vorderteil des Verschlußkastens weg nach innen. Der Federarm taucht in seiner Entriegelungsstellung in eine Ausnehmung in der Bodenplatte des Verschlußkastens ein.

Ausgehend von der eingangs beschriebenen Hyperextensionsorthese liegt der Erfindung die Aufgabe zugrunde, den genannten Verschlußkasten in seiner Bauhöhe zu verringern.

Ausgehend von der eingangs beschriebenen Hyperextensionsorthese wird diese Aufgabe erfindungsgemäß dadurch gelöst, daß der den Schließhaken tragende Federarm an seinem dem Schließhaken abgewandten, in Einschubrichtung des Taillenbandes vorn liegenden Ende mit einem oder mehreren Zapfen oder einer Feder in eine entsprechend geformte Ausnehmung wie z. B. eine Bohrung oder Nut des die Bodenabdeckung des Verschlußkastens bildenden Seitensteges formschlüssig eingreift, daß der Schließhaken von dem genannten Seitensteg weg nach außen ragt, und daß der Federarm in seiner Entriegelungsstellung in eine Ausnehmung im Seitensteg eintaucht.

Ein wesentlicher Vorteil der erfindungsgemäßen Orthese bzw. ihres Verschlußkastens liegt in der Verringerung der Bauhöhe des Verschlußkastens. Erfindungsgemäß wird dies in erster Linie dadurch erreicht, daß die Materialstärke des Seitensteges für den bei seiner Verschwenkung in eine Ausnehmung dieses Seitensteges eintauchenden Federarm zur Verfügung steht.

Für die Funktion ist vorteilhaft, daß die von dem Taillenband auf den Schließhaken ausgeübten Zugkräfte über das Federarmbauteil und dessen in eine Nut des Seitensteges formschlüssig eintauchende Feder unmittelbar in den vorzugsweise aus Metall bestehenden Seitensteg eingeleitet werden, also nicht durch auf Schwerkraft beanspruchte Verbindungsniete oder dergl. aufgenommen werden müssen.

Der Bedienungskomfort zur Entriegelung des Taillenbandes im Verschlußkasten wird erfindungsgemäß dadurch verbessert, daß das aus dem Verschlußkasten herausragende, manuell zu beaufschlagende Ende der beiden Schieber als leicht nach innen gewölbtes, anatomisch geformtes Griffstück ausgebildet ist.

Um zu verhindern, daß das Taillenband unter Einwirkung der einwirkenden Zugkräfte von dem sich in seiner Verriegelungsstellung befindlichen Schließhaken abgezogen wird, ist es vorteilhaft, wenn die die von dem Taillenband auf den Schließhaken ausgeübten, der Einschubrichtung entgegengerichteten Zugkräfte von der als leicht hinterschnittene Schräge ausgebildeten Stirnkante des Schließhakens aufgenommen werden.

Weitere Merkmale der Erfindung sind Gegenstand der Unteransprüche und werden in Verbindung mit weiteren Vorteilen der Erfindung anhand eines Ausführungsbeispieles näher erläutert.

In der Zeichnung ist eine als Beispiel dienende Ausführungsform der Erfindung dargestellt. Es zeigen:
- Figur 1: in schematischer Darstellung das Vorderteil einer Hyperextensionsorthese mit dem festzulegenden Ende eines Taillenbandes;
- Figur 2: in vergrößertem Maßstab in Draufsicht ein Rahmenbauteil eines Verschlußkastens zur Festlegung des freien Endes des Taillenbandes;
- Figur 3: in Draufsicht ein von dem Rahmenbauteil gemäß Figur 2 aufzunehmendes Federarmbauteil;
- Figur 4: in gegenüber den Figuren 2 und 3 vergrößertem Maßstab einen lotrechten Längsschnitt durch den montierten Verschlußkasten;
- Figur 5: in Draufsicht das Rahmenbauteil gemäß Figur 2 mit eingesetztem Federarmbauteil gemäß Figur 3 und eingebauten Schiebern und
- Figur 6: einen Schnitt gemäß der Linie A-A in Figur 5.

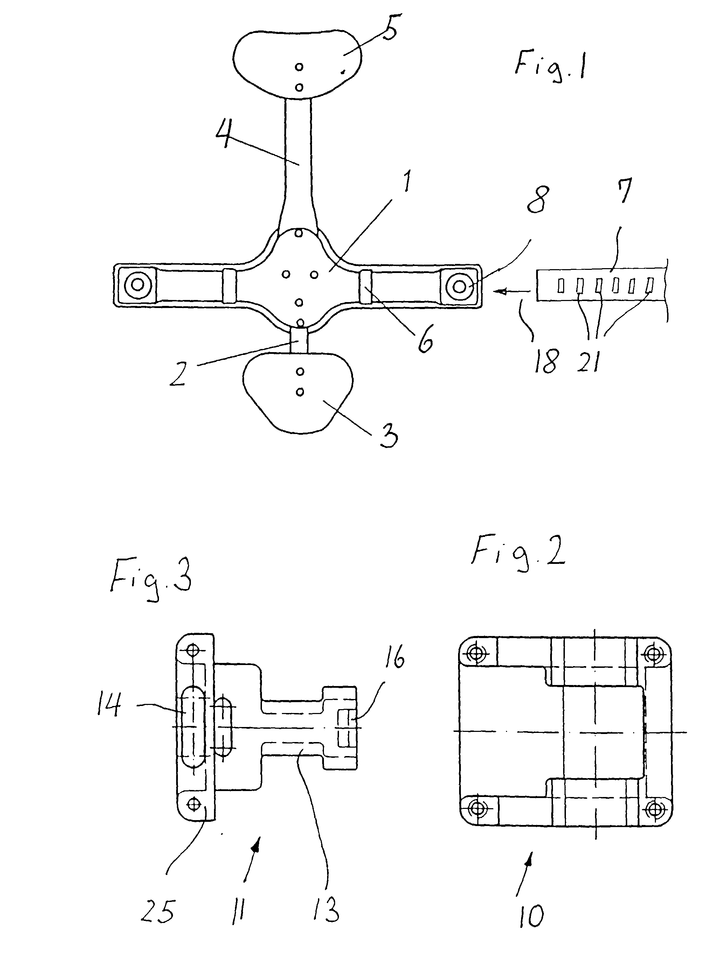

Das in Figur 1 dargestellte Vorderteil einer Hyperextensionsorthese setzt sich zusammen aus einer Basisplatte 1, von der aus sich nach unten ein Bauchstab 2 erstreckt, der an seinem unteren Ende eine Symphysenpelotte 3 trägt. Von der Basisplatte 1 nach oben erstreckt sich ein Bruststab 4, der an seinem oberen Ende eine Sternalpelotte 5 trägt. Von der Basisplatte 1 erstrecken sich nach jeder Seite Seitenstege 6, an deren Enden die Enden je eines Taillenbandes 7 befestigt sind, das an eine in der Zeichnung nicht näher dargestellte Rückenpelotte angeschlossen ist. Das in Figur 1 am rechten Seitensteg 6 schematisch dargestellte Verschlußelement 8 für die Festlegung des Taillenbandes 7 ist Gegenstand der Erfindung.

Das Verschlußelement 8 ist als Verschlußkasten 9 ausgebildet, dessen Bodenabdeckung durch den Seitensteg 6 gebildet wird. Die Darstellung ist gegenüber der der Figur 1 um 180° verdreht.

Der Verschlußkasten 9 umfaßt ein Rahmenbauteil 10 gemäß Figur 2, ein in diesem Rahmenbauteil 10 angeordnetes Federarmbauteil 11 gemäß Figur 3 sowie zwei Schieber 12.

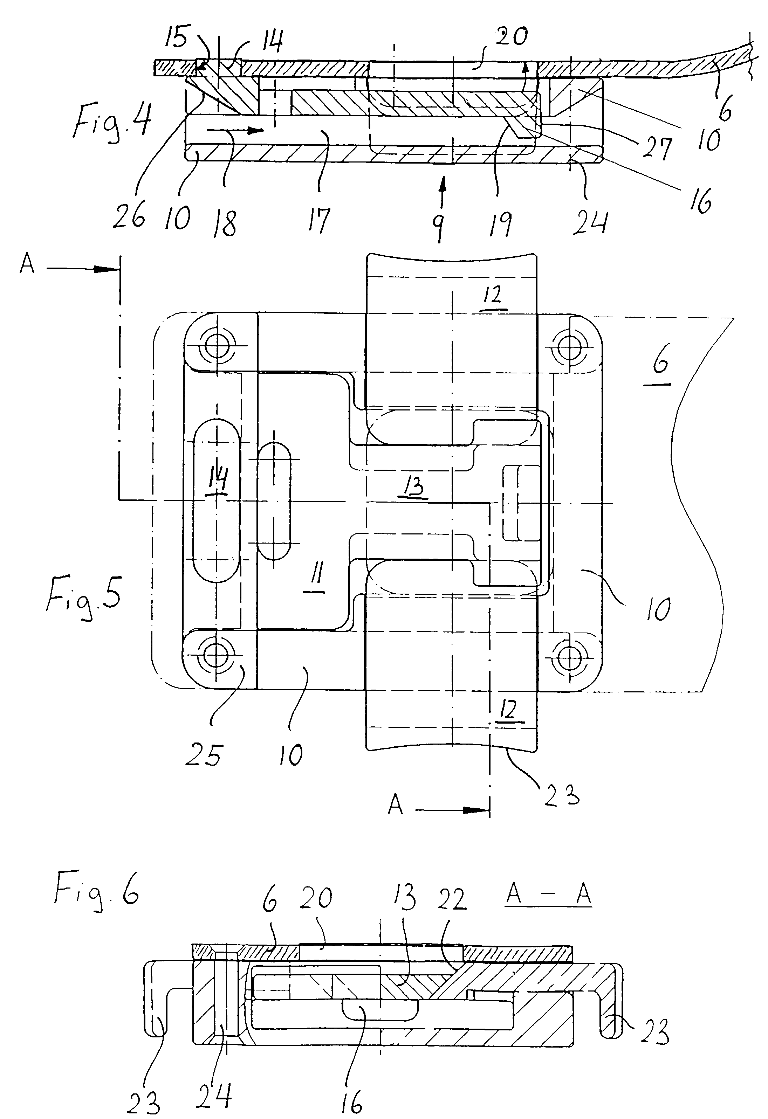

Das in Figur 3 in Draufsicht und in Figur 4 im Längsschnitt dargestellte Federarmbauteil 11 weist einen federelastisch ausgebildeten Federarm 13 auf, der an seinem einen Ende mit einer Feder 14 bestückt ist, die in eine ihr angepaßte Nut 15 des vorzugsweise aus Aluminium bestehenden Seitensteges 6 formschlüssig eingreift. Das andere Ende des Federarms 13 ist als Schließhaken 16 ausgebildet, der in der in Figur 4 dargestellten Ruhestellung in einen Einschubschlitz 17 ragt, der sich durch den Verschlußkasten 9 hindurch erstreckt (siehe insbesondere Figur 4), in den das in Figur 1 dargestellte freie Ende des Taillenbandes 7 zu dessen Verriegelung in Richtung des Pfeiles 18 eingeschoben wird. Der in Einschubrichtung 18 vorlaufende Stirnrand des Taillenbandes 7 läuft gegen eine Hubschräge 19 des Schließhakens 16 an und drückt dadurch diesen und damit auch den ihn tragenden Federarm 13 in Richtung des in Figur 4 eingezeichneten Pfeiles nach oben, wobei der Federarm 11 in eine Ausnehmung 20 im Seitensteg 6 eintaucht. Das Taillenband 7 wird so weit eingeschoben, bis eine die gewünschte Taillenbandlänge definierende, im Taillenband 7 vorgesehene Rastausnehmung 21 unterhalb von dem Schließhaken 16 liegt, der daraufhin unter der federelastischen Wirkung des Federarmes 13 wieder in die in Figur 4 dargestellte Verriegelungsstellung verschwenkt.

Zur Entriegelung des Schließhakens 16 sind zwei manuell gegeneinander drückbare, quer zum Einschubschlitz 17 im Verschlußkasten 9 verschiebbar gelagerte Schieber 12 vorgesehen, die mit einem durch ihr innenliegendes Ende gebildeten Hubschräge 22 das den Schließhaken 16 tragende Ende des Federarms 13 untergreifen und diesen beim weiteren Zusammendrücken der Schieber 12 nach oben in seine Entriegelungsstellung drücken, in der der Federarm 13 - wie vorstehend erläutert-in die Ausnehmung 20 des Seitensteges 6 eintaucht. Zur Erleichterung der Bedienung ist das aus dem Verschlußkasten 9 herausragende Ende jedes Schiebers 12 als leicht nach innen gewölbtes, anatomisch geformtes Griffstück 23 ausgebildet.

Der Verschlußkasten 9 ist mit dem seine Bodenabdeckung bildenden Seitensteg 6 bei 24 vernietet, wobei die beiden zur Feder 14 benachbarten Niete zugleich auch durch ein Haltestück 25 des Federarmbauteils 11 geführt sind. Das Rahmenbauteil 10 weist an seinem in Einschubrichtung 18 vorn liegenden Ende eine das Einführen des Taillenbandes 7 erleichternde Einschubschräge 26 auf.

Zur Sicherung der Verriegelungsstellung des Schließhakens 16 auch unter Last, d.h. unter Einwirkung von auf ihn durch das Taillenband 7 ausgeübten Zugkräften, weist der Schließhaken 16 stirnseitig eine leichte Schräge 27 auf, die bei einer dem Pfeil 18 entgegengerichteten Zugkraft des Taillenbandes 7 dieses in dichte Anlage an den Federarm 11 zieht und dadurch die Verriegelung über den Schließhaken 16 sicherstellt.

Rahmenbauteil 10, Federarmbauteil 11 sowie Schieber 12 sind vorzugsweise aus Kunststoff ausgebildet; das Taillenband 7 besteht vorzugsweise aus einem biegeelastischen Kunststoff.

## Patentansprüche

1. Hyperextensionsorthese mit einem Verschlußkasten (9), der an dem freien Ende eines sich von einer Basisplatte (1) zur Seite erstreckenden Seitensteges (6) befestigt ist und das eine Ende eines mit einer Rückenpelotte verbundenen Taillenbandes (7) selbsttätig verriegelnd aufnimmt, wobei der Verschlußkasten (9) einen das eine Ende eines Federarmes (13) bildenden Schließhaken (16) aufweist, der beim Einschieben des Taillenbandes (7) in einen sich durch den Verschlußkasten (9) hindurch erstreckenden Einschubschlitz (17) unter Federwirkung selbsttätig in eine der Rastausnehmungen (21) im Taillenband (7) eingreift und durch manuelles Zusammendrücken zweier quer zum Einschubschlitz (17) verschiebbar im Verschlußkasten (9) gelagerter Schieber (12) gegen die Federwirkung entriegelbar ist, **dadurch gekennzeichnet,** daß der den Schließhaken (16) tragende Federarm (13) an seinem dem Schließhaken (16) abgewandten, in Einschubrichtung (18) des Taillenbandes (7) vorn liegenden Ende mit einem oder mehreren Zapfen oder einer Feder (14) in eine entsprechend geformte Ausnehmung wie z. B. eine Bohrung oder Nut (15) des die Bodenabdeckung des Verschlußkastens (9) bildenden Seitensteges (6) formschlüssig eingreift,
daß der Schließhaken (16) von dem genannten Seitensteg (6) weg nach außen ragt, und
daß der Federarm (13) in seiner Entriegelungsstellung in eine Ausnehmung (20) im Seitensteg (6) eintaucht.

2. Hyperextensionsorthese nach Anspruch 1, **dadurch gekennzeichnet,** daß der Verschlußkasten (9) mit dem seine Bodenabdeckung bildenden Seitensteg (6) vernietet (24) ist.

3. Hyperextensionsorthese nach Anspruch 1 oder 2, **dadurch gekennzeichnet,** daß das aus dem Verschlußkasten (9) herausragende, manuell zu beaufschlagende Ende der beiden Schieber (12) als leicht nach innen gewölbtes, anatomisch geformtes Griffstück (23) ausgebildet ist.

4. Hyperextensionsorthese nach Anspruch 1, 2 oder 3, **dadurch gekennzeichnet,** daß die die von dem Taillenband (7) auf den Schließhaken (16) ausgeübten, der Einschubrichtung (80) entgegengerichteten Zugkräfte von der als leicht hinterschnittene Schräge (27) ausgebildeten Stirnkante des Schließhakens (16) aufgenommen werden.

5. Hyperextensionsorthese nach einem der vorhergehenden Ansprüche, **dadurch gekennzeichnet,** daß der Seitensteg (6) aus Aluminium besteht.

## Claims

1. Hyperextension orthesis with a closure box (9) which is fastened to the free end of a lateral strap (6) extending sideways from a base plate (1) and which receives, in automatically locking manner, one end of a waistband (7) which is connected to a dorsal pad, whereby the closure box (9) comprises a clasp (16) which forms one end of a spring arm (13) and which, when the waistband (7) is inserted into an insertion slot (17) extending through the closure box (9), automatically engages, under spring action, in one of the latching recesses (21) in the waistband (7) and is capable of being unlocked, contrary to the action of the spring, by manual compression of two slides (12) which are displaceably supported in the closure box (9) at right angles to the insertion slot (17),
**characterised in that** the spring arm (13) bearing the clasp (16) engages positively, at its end facing away from the clasp (16) and situated at the front in the insertion direction (18) of the waistband (7) with one or more lugs or with a spring (14) in a correspondingly shaped recess such as, for example, a bore or groove (15) in the lateral strap (6) forming the bottom cover of the closure box (9),
in that the clasp (16) projects outwards away from the aforementioned lateral strap (6), and
in that the spring arm (13) in its unlocking position protrudes into a recess (20) in the lateral strap (6).

2. Hyperextension orthesis according to Claim 1, **characterised in that** the closure box (9) is riveted (24) to the lateral strap (6) forming its bottom cover.

3. Hyperextension orthesis according to Claim 1 or 2, **characterised in that** the end of the two slides (12) which projects out of the closure box (9) and which is to be operated manually takes the form of a gripping piece (23) which is slightly curved inwards and shaped anatomically.

4. Hyperextension orthesis according to Claim 1, 2 or 3, **characterised in that** the tensile forces exerted by the waistband (7) on the clasp (16) and directed contrary to the insertion direction (18) are accommodated by the front edge, taking the form of a slightly undercut bevel (27), of the clasp (16).

5. Hyperextension orthesis according to one of the preceding claims, **characterised in that** the lateral strap (6) consists of aluminium.

## Revendications

1. Orthèse d'hyperextension comprenant un boîtier de bouclage (9) qui est fixé à l'extrémité libre d'une aile latérale (6) s'étendant vers le côté à partir d'une plaque de base (1) et qui reçoit avec verrouillage automatique une extrémité d'une bande de ceinture (7) reliée à un coussin dorsal, le boîtier de bouclage (9) présentant un crochet de fermeture (16) formant l'une des extrémités d'un bras à ressort (13), lequel crochet (16), lors de l'insertion de la bande de ceinture (7) dans une fente d'insertion (17) traversant le boîtier de bouclage (9), s'engage automatiquement, par action de ressort, dans l'un des évidements d'accrochage (21) prévus dans la bande de ceinture (7) et peut être déverrouillé contre l'action de ressort en pressant manuellement l'un vers l'autre deux poussoirs (12) supportés dans le boîtier de bouclage (9) de façon à pouvoir coulisser transversalement à la fente d'insertion (17), caractérisée en ce qu'à son extrémité opposée au crochet de fermeture (16), située en avant dans la direction d'introduction (18) de la bande de ceinture (7), le bras à ressort (13) portant le crochet de fermeture (16) comporte un ou plusieurs tenons ou une clavette (14) par les(s)quel(s) ou laquelle le bras (13) s'engage avec verrouillage par complémentarité de forme dans un évidement formé de manière correspondante, comme par exemple un alésage ou une rainure (15) de l'aile latérale (6) formant la couverture de fond du boîtier de bouclage,
en ce que le crochet de fermeture (16) fait saillie vers l'extérieur en s'éloignant de l'aile latérale précitée (6), et
en ce que dans sa position de déverrouillage, le bras à ressort (13) s'efface dans un évidement (20) dans l'aile latérale (6) .

2. Orthèse d'hyperextension selon la revendication 1, caractérisée en ce que le boîtier de bouclage (9) est riveté (24) avec l'aile latérale (6) formant sa couverture de fond.

3. Orthèse d'hyperextension selon la revendication 1 ou 2, caractérisée en ce que l'extrémité destinée à l'action manuelle, saillant hors du boîtier de bouclage (9), des deux poussoirs (12), est réalisée en pièce de préhension (23) conformée anatomiquement, légèrement incurvée vers l'intérieur.

4. Orthèse d'hyperextension selon la revendication 1, 2 ou 3, caractérisée en ce que les forces de traction opposées à la direction d'introduction (18), exercées par la bande de ceinture (7) sur le crochet de fermeture (16) sont reprises par le bord frontal du crochet de fermeture (16) réalisé sous la forme d'un biseau en légère contre-dépouille (27) .

5. Orthèse d'hyperextension selon l'une des revendications précédentes, caractérisée en ce que l'aile latérale (6) est constituée d'aluminium.
